# EUROPÄISCHE PATENTSCHRIFT

(11) **EP 1 871 441 B1**
(45) Veröffentlichungstag und Bekanntmachung des Hinweises auf die Patenterteilung: **05.11.2014**
(21) Anmeldenummer: 06743298.9
(22) Anmeldetag: 13.04.2006
(51) Int. Cl.: A61M 1/10

(54) **BLUTPUMPE**
BLOOD PUMP
POMPE A SANG

(30) Priorität: 16.04.2005 DE 102005017546
(43) Veröffentlichungstag der Anmeldung: 02.01.2008
(73) Patentinhaber: Abiomed Europe GmbH, 52074 Aachen (DE)
(72) Erfinder: SIESS, Thorsten, 52146 Würselen (DE); DAMEN, Rainer, 52080 Aachen (DE)
(74) Vertreter: Von Kreisler Selting Werner - Partnerschaft von Patentanwälten und Rechtsanwälten mbB
(86) Internationale Anmeldenummer: PCT/EP2006/061582
(87) Internationale Veröffentlichungsnummer: WO 2006/111503

(56) Entgegenhaltungen:
- WO-A-03/105669
- US-A- 4 846 152
- US-A- 6 139 487
- HIROFUMI ANAI ET AL: "AN APPROACH TO REDUCING HEMOLYSIS IN AN AXIAL-FLOW BLOOD PUMP" ASAIO JOURNAL, LIPPINCOTT WILLIAMS & WILKINS / ASAIO, HAGERSTOWN, MD, US, Bd. 41, Nr. 3, 1. Juli 1995 (1995-07-01), Seiten 771-774, XP000542964 ISSN: 1058-2916

## Beschreibung

Die Erfindung betrifft eine intrakardiale Blutpumpe entsprechend dem Oberbegriff des Patentanspruchs 1, die eine Auslegungsdrehzahl mit minimaler Ablösung und Verwirbelung der Blutströmung aufweist.

In EP 0 961 621 B1 (Impella) ist eine intravasale Blutpumpe beschrieben, die durch das Gefäßsystem in den Körper eingeführt und im Herzen oder an einer anderen Stelle, an der Blut gepumpt werden soll, platziert wird. Die Blutpumpe weist ein Gehäuse mit einem Durchmesser von 5,4 bis 6,4 mm auf, das den Stator eines Motors bildet. Der Rotor ist mit einem Impeller verbunden, der in einem Pumpenring rotiert. Diese sehr kleinformatige Blutpumpe hat eine relativ hohe Drehzahl in der Größenordnung von 30.000 U/min.

In EP 0 925 080 B1 (Impella) ist ein Verfahren zur Steuerung der Drehzahl einer Blutpumpe beschrieben, bei dem die Blutpumpe Drucksensoren aufweist, um die an der Blutpumpe herrschende Druckdifferenz zu ermitteln. In Abhängigkeit von den Signalen der Drucksensoren, wird die Motordrehzahl gesteuert. Dadurch wird ein gewünschter Volumenstrom erzeugt, um die gewünschte Pumpwirkung sicherzustellen.

In US 6,139,487 A ist eine Pumpvorrichtung mit zwei intrakardialen Blutpumpen beschrieben, die durch eine gemeinsame Steuereinheit gesteuert sind, so dass die Volumenströme beider Blutpumpen einander angeglichen werden.

Kontinuierlich fördernde rotatorische Blutpumpen haben eine sogenannte Auslegungsdrehzahl. Der das Blut vortreibende Impeller ist so geformt, dass bei der Auslegungsdrehzahl die Strömungsabrisse an den Impeller-Flügeln minimiert sind, oder idealerweise Strömungsabrisse überhaupt nicht auftreten. Dadurch wird eine turbulenzarme Strömung erreicht. Strömungsabrisse und Turbulenzen bewirken die Gefahr von Thrombenbildung, wobei Blut akkumuliert und an dem Impeller oder an anderen Stellen des Blutsystems anwächst. Dadurch wird zunächst die Funktion des Impellers verschlechtert. Außerdem besteht die Gefahr von Verstopfungen des Blutsystems.

Der Erfindung liegt die Aufgabe zugrunde, eine rotatorische Blutpumpe anzugeben, die permanent eine Blutförderung mit hoher Effektivität sicherstellt und es auch erlaubt, die Blutpumpe zeitweise unterhalb der Auslegungsdrehzahl zu betreiben.

Die erfindungsgemäße Blutpumpe weist die Merkmale des Patentanspruchs 1 auf.

Die Erfindung basiert auf der Erkenntnis, dass eine Thrombenbildung am Impeller dadurch vermieden werden kann, dass mindestens zeitweise die Auslegungsdrehzahl eingenommen wird. Im Übrigen besteht aber die Möglichkeit, die Blutpumpe gefahrlos mit geringerer Drehzahl zu betreiben. Dadurch, dass jeweils nach einer gewissen Zeit des Betriebes mit geringer Drehzahl die Drehzahl auf die Auslegungsdrehzahl erhöht wird, werden Ansätze von Thrombenbildung am Impeller reformiert. Es hat sich gezeigt, dass ein nur kurzzeitiger Betrieb mit der Auslegungsdrehzahl ausreicht, um Ansätze von Thromben vom Impeller abzulösen, so dass ein Aufbau von Thromben vermieden wird. Die Erfindung erlaubt es, den Volumenstrom bzw. die Fördermenge unterhalb der Auslegungsdrehzahl zu wählen und erforderlichenfalls auch zu variieren, ohne dass ein ständiger Betrieb mit der hohen Auslegungsdrehzahl erforderlich wäre.

Die Erhöhung von der niedrigen Drehzahl auf die Auslegungsdrehzahl erfolgt vorzugsweise mit einer Beschleunigung, die größer ist als 3.000 s⁻². Dadurch wird ein kurzzeitiger und rapider Anstieg der Pumpendrehzahl erreicht. Dies ist wegen der geringen Masse der Blutpumpe, die als intravasale oder parakardiale Blutpumpe ausgebildet ist, möglich.

Die Steuerung der Drehzahl der Blutpumpe erfolgt vorzugsweise durch Vorgabe einer Erregerfrequenz. Bei dem Rotor handelt es sich um einen schleifringlosen Gleichstrommotor, der im Stator zyklisch erregte Wicklungen aufweist und im Rotor mit Permanentmagneten versehen ist. Ein entfernt von dem Rotor angeordnetes Steuergerät liefert die Betriebsfrequenz für den Motor. Der Motor ist mit dem Steuergerät durch eine elektrische Leitung verbunden, die durch einen flexiblen Katheter hindurchführt.

Die Verlangsamung der Drehzahl bedarf einer Energiezufuhr. Sie erfolgt vorzugsweise durch ungebremstes natürliches Abklingen (Dämpfung durch den antreibenden Impeller), wobei die Verlangsamung ebenfalls größer ist als 3.000 s⁻² und somit innerhalb sehr kurzer Zeit erfolgt.

Im Rahmen der Erfindung ist es möglich, die rotatorische Blutpumpe nach einer ersten Verfahrensvariante zu betreiben, bei der periodisch Impulse mit der Auslegungsdrehzahl ausgeführt werden, und nach einer zweiten Variante, bei der die Beschleunigung und Verlangsamung fremdgesteuert durchgeführt werden.

Bei der ersten Variante wird die Förderung hauptsächlich durch die niedrige Drehzahl bestimmt und das Fördervolumen wird durch die kurzzeitigen Erhöhungen auf die Auslegungsdrehzahl nur unwesentlich beeinflusst. Dies hat zur Folge, dass die niedrige Drehzahl entsprechend dem Bedarf des Patienten eingestellt werden kann. Wenn sich das Herz des Patienten aufgrund der Unterstützung durch die Blutpumpe erholt hat und nunmehr weniger Unterstützung braucht, kann eine entsprechende niedrige Drehzahl der Blutpumpe gewählt werden, wobei die Entstehung von Thromben durch kurzzeitige Nadelimpulse vermieden wird, in denen die Drehzahl auf die Auslegungsdrehzahl erhöht wird. Diese Drehzahlerhöhungen beeinflussen die gesamte Fördermenge der Blutpumpe nur unwesentlich.

Bei der zweiten Variante kann mit der Blutpumpe, die eigentlich für eine kontinuierliche Förderung vorgesehen ist, eine pulsatile Herzunterstützung erfolgen. Der Pumpfunktion der Blutpumpe wird die natürliche Pumpfunktion des Herzens überlagert. Hieraus ergibt sich ein periodisch veränderlicher Strom, wobei die periodische Veränderung durch die Pumpfunktion des Herzens hervorgerufen wird, durch die die Blutpumpe zeitweilig unterstützt wird, nämlich während der Systole, und zeitweilig gehemmt wird, nämlich während der Diastole. Die Erfindung ermöglicht es, durch Überwachung des Motorstroms die Zeiten hoher Pumpendrehzahl und niedriger Pumpendrehzahl mit der Pumpfunktion des Herzens zu synchronisieren bzw. die Herzunterstützung phasenversetzt zur Herzfunktion zu triggern, ohne dass eine EKG-Ableitung erforderlich wäre.

Die Erfindung ist insbesondere für die Rechtsherzunterstützung geeignet, bei der die Saugöffnung der Pumpe im rechten Atrium platziert wird, während die Pumpe in die Pulmonalarterie fördert.

Im Folgenden werden unter Bezugnahme auf die Zeichnungen Ausführungsbeispiele der Erfindung näher erläutert.

Es zeigen:
- Figur 1: eine schematische Darstellung des Aufbaus der Blutpumpe,
- Figur 2: in vergrößertem Maßstab die Strömungsverhältnisse an einem Flügel des Impellers der Pumpe,
- Figur 3: den zeitlichen Verlauf der Pumpendrehzahl bei periodischer Steuerung,
- Figur 4: den zeitlichen Verlauf der Pumpendrehzahl bei einem Pumpenbetrieb, der mit der Herzfunktion synchronisiert ist, und
- Figur 5: eine schematische Darstellung des Pumpenantriebs und des Sensors zur Ermittlung des Motorstromes.

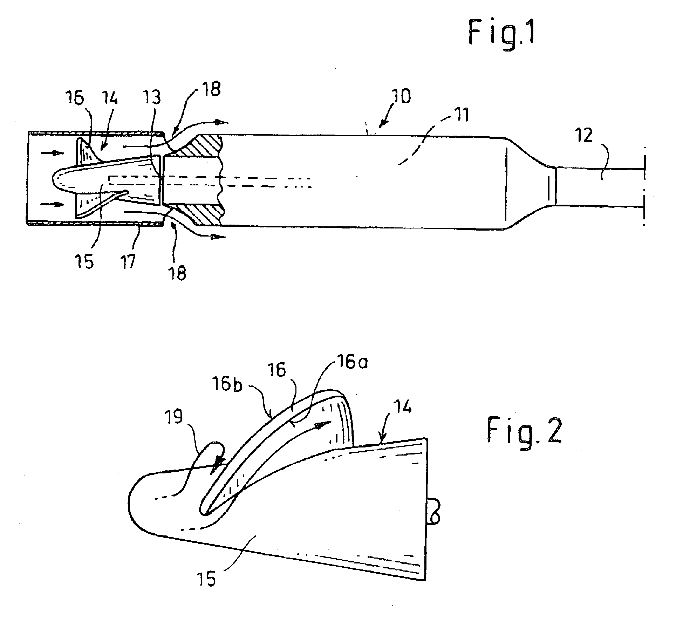

In der Figur 1 ist eine rotatorische Blutpumpe 10 zur Herzunterstützung dargestellt. Die Blutpumpe ist hier eine intravasale Blutpumpe, deren maximaler Außendurchmesser an keiner Stelle größer ist als 7 mm. Die Blutpumpe kann aber auch als intrakardiale Blutpumpe ausgebildet sein, die durch eine Inzisionsstelle des Herzens hindurch geführt wird. Eine derartige Blutpumpe kann einen etwas größeren Durchmesser haben. Ferner kann die Pumpe parakardial (um das Herz herum) platziert werden und über zwei Inzisionsstellen Blut vor oder aus dem Herzen entnehmen und hinter dem Herzen durch die zweite Inzisionsstelle wieder zuführen.

Die Blutpumpe 10 weist einen langgestreckten zylindrischen Motor 11 auf, an dessen proximales Ende sich ein flexibler Katheter 12 anschließt. Durch den Katheter 12 gehen (nicht dargestellte) elektrische Leitungen hindurch, die zu einem Steuergerät führen.

Die Welle 13 des Motors 11 trägt einen Impeller 14 mit einer zum distalen Ende hin spitz zulaufenden Nabe 15, von der schraubenförmige Flügel 16 abstehen. Der Impeller 14 ist von einem länglichen Ring 17 umgeben, der etwa den gleichen Außendurchmesser hat wie der Motor 11. Zwischen dem Motor 11 und dem Ring 17 befinden sich Ausströmöffnungen 18. Durch Rotation des Impellers 14 saugt dieser axial Blut an und fördert es in Richtung auf den Motor 11. Das Blut verlässt die Blutpumpe durch die Öffnungen 18, um anschließend an dem Motor 11 entlang zu strömen. Die in Figur 1 dargestellte Pumpe kann ebenfalls so strömungstechnisch ausgelegt werden, dass eine umgekehrte Flussrichtung erreicht wird.

Figur 2 zeigt eine schematische vergrößerte Darstellung eines Teils des Impellers 14. Von der Nabe 15 steht ein Flügel 16 ab, der nach Art eines Flugzeugflügels gebogen ist. Infolge der Strömung entsteht an der konkaven Innenseite 16a ein positiver Druck und entlang der konvexen Oberseite 16b entsteht ein Unterdruck oder negativer Druck. Die Flügel 16 sind so gestaltet, dass bei einer Auslegungsdrehzahl eine abrissfreie Strömung entlang der Flügel entsteht. Bei dem vorliegenden Ausführungsbeispiel beträgt die Auslegungsdrehzahl 30.000 U/min. Bei niedrigeren Drehzahlen entstehen Strömungsabrisse und Wirbel 19, die eine Blutschädigung durch Entstehung von Thromben bewirken können. Die Thromben wachsen auf der Oberfläche des Flügels 16 und beeinträchtigen die Pumpenströmung, wodurch die Thrombenbildung noch weiter verstärkt wird.

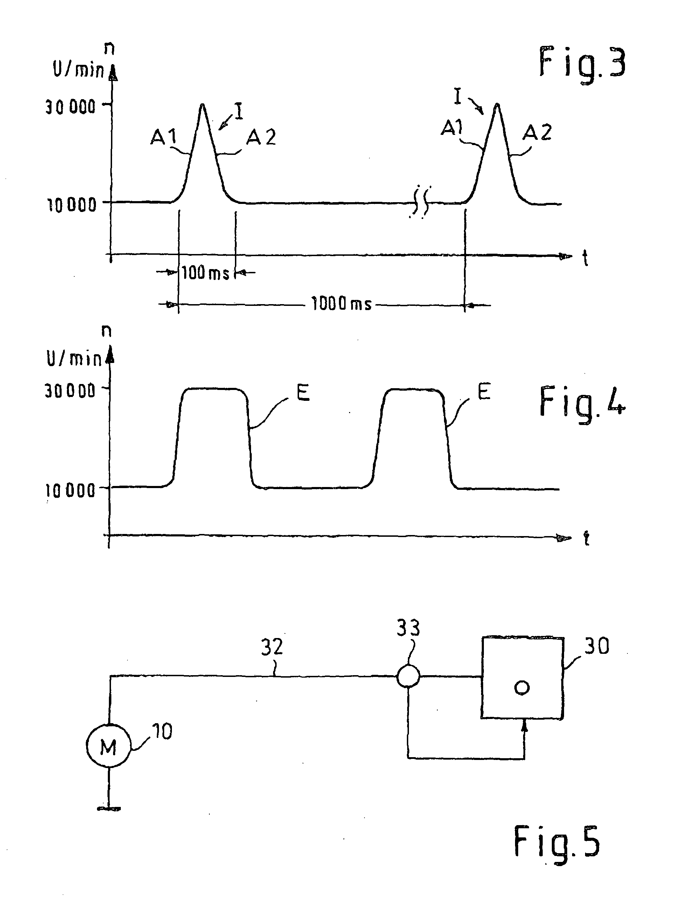

Figur 3 zeigt ein Diagramm eines zeitlichen Verlaufs der Drehzahl n des Motors 11, wobei längs der Abszisse die Zeit t und längs der Ordinate die Drehzahl n in Umdrehungen pro Minute aufgetragen sind. Die Drehzahl kann durch entsprechende Einstellung des Steuergerätes variiert werden. Bei dem vorliegenden Beispiel ist angenommen, dass die Drehzahl generell 10.000 U/min betragen soll. Dieser Wert entspricht beispielhaft der Fördermenge, die der Patient zur Herzunterstützung in der Entwöhnungsphase benötigt.

Die kontinuierliche Drehzahl von 10.000 U/min wird unterbrochen durch periodisch auftretende Impulse I, in denen die Drehzahl kurzzeitig auf die Auslegungsdrehzahl von 30.000 U/min ansteigt. Die Anstiegsflanke A1 des Impulses I hat eine Steigung von mehr als 3.000 s⁻². In ihr steigt die Drehzahl des Motors kontinuierlich von dem niedrigen Wert von 10.000 U/min auf die Auslegungsdrehzahl von 30.000 U/min an. Dieser Frequenzanstieg wird durch das Steuergerät erzeugt. Nach Erreichen der Auslegungsdrehzahl klingt die Drehzahl in einer Abstiegsflanke A2 ab. Dieser Abfall erfolgt ohne Zuführung von Bremsenergie oder anderweitiger externer Energie durch Unterlassung der Zufuhr von Antriebsenergie. Auch das Abklingen erfolgt mit einer Verzögerung von mehr als 3.000 s⁻². Das Abklingen wird beendet, wenn die Drehzahl wieder den unteren Wert erreicht hat, der anschließend beibehalten wird. Die Dauer der Impulse I beträgt bei dem vorliegenden Ausführungsbeispiel etwa 100 ms und die Periodendauer, also die Dauer eines Impulses und einer anschließenden Impulslücke, beträgt etwa 1.000 ms.

Die Impulse I sind Nadelimpulse mit im Verhältnis zur Periodendauer kurzer Impulsbreite. Daher ist auch die zusätzlich Förderleistung, die durch die Impulse I erbracht wird, im Verhältnis zu der Grund-Förderleistung bei 10.000 U/min klein. Diese Grund-Förderleistung wird durch die Impulse I also wenig beeinflusst. Die Impulse I haben die Wirkung, dass trotz eines geringen Flusses Thrombenbildungen am Impeller selbst über Tage hinweg vermieden werden.

Figur 4 zeigt eine zweite Variante des erfindungsgemäßen Verfahrens. Bei dieser wird ebenfalls die Drehzahl n durch Impulse E vorübergehend erhöht, jedoch sind die Impulse E wesentlich breiter als die Impulse I nach der ersten Verfahrensvariante. Die Impulse E, in denen die Drehzahl vorübergehend erhöht wird, werden synchron mit der Herzfrequenz erzeugt, jedoch hierzu phasenversetzt. Figur 5 zeigt die Schaltung des Motors 11 und eines Steuergerätes 30, das die Frequenz und den Strom für den Motor 11 liefert. Das Steuergerät 30 ist mit dem Motor über eine Leitung 32 verbunden. Ein Sensor 33 misst den in der Leitung 32 fließenden Strom und teilt diesen dem Steuergerät 30 mit. Der Motorstrom ist abhängig von der Last des Motors. Die Last ändert sich über der Zeit entsprechend der natürlichen Pumpwirkung des Herzens, die der kontinuierlichen Pumpwirkung der Pumpe 10 überlagert ist. Aus dem zeitlichen Verlauf des Stromes, der von Sensor 33 gemessen wird, kann die Zeitsteuerung für die Erzeugung der Impulse E in Figur 4 abgeleitet werden. Dadurch wird eine EKG-Ableitung mit zusätzlichen Messelektroden vermieden. Die Pumpe benötigt keine externen Sensoren.

Gemäß Figur 4 erfolgt der Betrieb der Pumpe auch bei dieser Variante des Verfahrens in der Weise, dass entweder eine niedrige Drehzahl n oder die Auslegungsdrehzahl eingestellt ist. Die Beschleunigung der Pumpe und die Verlangsamung betragen auch hier mehr als 3.000 s⁻². Bei dieser Variante des Pumpenbetriebes wird mit der rotatorischen Blutpumpe ein pulsierender Pumpenbetrieb erzeugt, der mit der natürlichen Pumpfrequenz des Herzens abgestimmt ist.

## Patentansprüche

1. Intrakardiale Blutpumpe mit einem elektrischen Motor (11), der über einen Katheter (12) mit einem Steuergerät (30) verbunden ist, und einem von dem Motor (11) angetriebenen Impeller (14), wobei die Blutpumpe eine Auslegungsdrehzahl hat, bei der Strömungsabrisse an den Flügeln eines Impellers minimiert sind
**dadurch gekennzeichnet,**
**dass** das Steuergerät (30) die Pumpendrehzahl zwischen der Auslegungsdrehzahl und einer niedrigeren Drehzahl variiert.

2. Intrakardiale Blutpumpe nach Anspruch 1, **dadurch gekennzeichnet, dass** ein den Motorstrom messender Sensor (33) vorgesehen ist, und dass das Steuergerät (30) die Pumpendrehzahl in Abhängigkeit von den Signalen des Sensors (33) variiert.

3. Intrakardiale Blutpumpe nach Anspruch 1, **dadurch gekennzeichnet, dass** die Erhöhung von der niedrigen Drehzahl auf die Auslegungsdrehzahl mit einer Beschleunigung erfolgt, die größer ist als 3.000 s⁻².

4. Intrakardiale Blutpumpe nach Anspruch 1 oder 2, **dadurch gekennzeichnet, dass** die Verlangsamung von der Auslegungsdrehzahl auf die niedrige Drehzahl durch ungebremstes natürliches Abklingen erfolgt.

5. Intrakardiale Blutpumpe nach Anspruch 3, **dadurch gekennzeichnet, dass** die Verlangsamung größer ist als 3.000 s⁻².

6. Intrakardiale Blutpumpe nach einem der Ansprüche 1-4, **dadurch gekennzeichnet, dass** das Steuergerät periodisch Impulse (I) mit der Auslegungsdrehzahl erzeugt, wobei die Periodendauer mindestens fünf Mal so lang ist wie die Breite der Impulse (I).

7. Intrakardiale Blutpumpe nach einem der Ansprüche 1-4, **dadurch gekennzeichnet, dass** ein den Motorstrom der Blutpumpe (10) messender Sensor (33) vorgesehen ist und aus dem Motorstrom der Rhythmus einer überlagerten Pumpfunktion bestimmt wird, und dass der Betrieb der Blutpumpe mit der überlagerten Pumpfunktion synchronisiert wird.

## Claims

1. An intracardiac blood pump comprising an electric motor (11) connected with a controller (30) via a catheter (12), and an impeller (14) driven by said motor (11), the blood pump (10) having a design rotational speed at which minimal detachment and swirling of a blood flow occur,
**characterized in that**
said controller (30) varies a rotational speed of said pump between the design rotational speed and a lower rotational speed.

2. The intracardiac blood pump according to claim 1, **characterized in that** a sensor (33) measuring a motor current is provided, and the controller (30) varies the rotational speed of said pump in accordance with the signals supplied by said sensor (33).

3. The intracardiac blood pump according to claim 1, **characterized in that** the increase from the low rotational speed to the design rotational speed takes place at an acceleration of more than 3,000 s⁻².

4. The intracardiac blood pump according to claim 1 or 2, **characterized in that** the deceleration from the design rotational speed to the low rotational speed takes place by a non-braked natural slowdown.

5. The intracardiac blood pump according to claim 3, **characterized in that** the deceleration exceeds 3,000 s⁻².

6. The intracardiac blood pump according to any one of claims 1-4, **characterized in that** periodic pulses (I) are produced at the design rotational speed, with a pulse cycle time being at least five times longer than a width of the pulses (I).

7. The intracardiac blood pump according to any one of claims 1-4, **characterized in that** a sensor (33) is provided for measuring the motor current of the blood pump (10), and a frequency of a superimposed pumping function is determined from the motor current, and the operation of the blood pump is synchronized with the superimposed pumping function.

## Revendications

1. Pompe à sang intracardiaque comprenant un moteur électrique (11) qui est relié à un appareil de commande (30) par un cathéter (12), et un rotor (14) entraîné par le moteur (11), la pompe à sang ayant une vitesse de rotation normale à laquelle les décrochages de l'écoulement sur les pales du rotor sont réduites à un minimum,
**caractérisée**
**en ce que** l'appareil de commande (30) fait varier la vitesse de rotation de la pompe entre la vitesse de rotation normale et une vitesse de rotation plus lente.

2. Pompe à sang intracardiaque selon la revendication 1 **caractérisée en ce qu'**il est prévu un capteur de mesure du courant du moteur (33) et **en ce que** l'appareil de commande (30) fait varier la vitesse de rotation de la pompe en fonction des signaux du capteur (33).

3. Pompe à sang intracardiaque selon la revendication 1, **caractérisée en ce que** l'élévation de la vitesse de rotation, de la vitesse de rotation lente à la vitesse de rotation normale, s'effectue avec une accélération qui est de plus de 3000 s⁻².

4. Pompe à sang intracardiaque selon la revendication 1 ou 2, **caractérisée en ce que** le ralentissement de la vitesse de rotation, de la vitesse de rotation normale à la vitesse de rotation lente, s'effectue par décroissance naturelle non freinée.

5. Pompe à sang intracardiaque selon la revendication 3, **caractérisée en ce que** le ralentissement est de plus de 3000 s⁻².

6. Pompe à sang intracardiaque selon l'une des revendications 1 à 4, **caractérisée en ce que** l'appareil de commande produit des impulsions périodiques (I) avec la vitesse de rotation normale, la durée de la période étant d'au moins cinq fois la largeur des impulsions (I).

7. Pompe à sang intracardiaque selon l'une des revendications 1 à 4, **caractérisée en ce qu'**il est prévu un capteur (33) qui mesure le courant du moteur de la pompe à sang (10) et que le rythme d'une fonction de pompe superposée est déterminé à partir du courant du moteur et **en ce que** le fonctionnement de la pompe à sang est synchronisé avec la fonction de pompe superposée.
